# EUROPEAN PATENT APPLICATION

(11) **EP 4 079 221 A1**
(43) Date of publication of application: **26.10.2022**
(21) Application number: 21169705.7
(22) Date of filing: 21.04.2021
(51) Int. Cl.: A61B 5/08, A61B 5/00, G16H 20/00, G16H 50/00

(54) **SYSTEM FOR IDENTIFYING A CONDITION OF A USER**

(71) Applicant: Oxypoint NV, 2018 Antwerpen (BE)
(72) Inventor: HENDRICKX, Philip, 2600 Berchem (BE); VAN STEENKISTE, Cedric, 2547 Lint (BE); PINTENS, Niels, 2900 Schoten (BE); VAN ROOST, Wouter, 1981 Hofstade (BE)
(74) Representative: DenK iP bv

(57) **Abstract**

A system adapted for deriving information regarding a condition of the user is described. The system comprises a processor configured for obtaining information related to the breathing cycle, wherein the processor furthermore is configured for comparing the derived information related to the breathing cycle of the user with predetermined breathing cycle information representative of different conditions and for identifying, based on said comparison, a condition of the user.

## Description

### Field of the invention

The invention relates to the medical field. More particularly, the present invention relates to systems and methods for determining breathing cycle related information for a user and/or for identifying a medical or other breathing cycle related condition of a user. Such systems and methods may make use of a gas valve system for delivering a gas to the user or components thereof.

### Background of the invention

To monitor the general status of a patient in a hospital, the nursing staff should measure the vital parameters for each patient at least 3 times a day. The 6 vital parameters are: temperature, blood pressure, breathing rhythm, heart rate, oxygen saturation and a consciousness score (in some more critical cases, urine and bowel movements are also monitored). It is on the basis of these measured values that, among other things, further therapy decisions are made. During the check-up, these parameters are often measured and written down manually, only to be archived in an electronic patient file. The frequency of measuring the vital parameters may vary on where the patient is staying (e.g. in hospital or under home care) or may vary depending on the state of the patient. Anyway, measuring, registering and digitizing parameters is very time and labour intensive.

The measurement of the respiratory rate (RR, also referred to as breath rhythm) - expressed in number of breaths per minute (bpm) - is usually done in hospital low care wards by counting the number of breaths of the patient for 20 seconds and afterwards multiplying the obtained value by 3 to a value per minute. At low care departments this is done by the nursing staff by looking at the movement of the chest. However, this is inaccurate and also highly biased. During this snapshot, the parameters of the patient involved are after all influenced by the so-called white coat effect and deviate from the actual values, purely by the presence of the hospital staff in the patient room. Due to the cumbersome and inaccurate nature, this procedure is sometimes skipped among less critical patients if the nursing staff finds that they are doing well. A variety of other techniques for measuring respiratory rate has been established over time. These techniques include manual or assisted counting, determination based on exhaled breathing, determination based on thoracic efforts, determination based on respiratory sounds, determination based on light measurements and determination based on indirect effects on the cardiovascular physiology or blood flow.

In the current situation there is a lack of therapy feedback, transparency and control (unless it concerns critical, high-care patients whose parameters are meticulously or even continuously monitored via expensive, cumbersome equipment). It is unknown what happens between 2 parameter rounds.

It is known in the art that the breathing parameters not only provide a view on the current medical condition of the patient, but may also be used to evaluate how the condition of the patient will evolve. For example, an increased respiratory rate may be an indication of acute myocardial infarction, of pneumonia, of a pulmonary embolism, of aspiration, of haemorrhage or of metabolic acidosis. A decreased respiratory rate may be an indication of a drug intoxication, of hypercapnia, of an intoxication or of a head injury.

Traditionally, as indicated above, typically up to six vital parameters are monitored to assess the patient amongst which respiratory rate, oxygen saturation, heart rate, blood pressure and temperature. These parameters typically are collected a number of times per day, by nurses performing different measurements. As indicated above, the frequency of monitoring these parameters may depend on the location of the patient (hospital or home care, as well as the patient's condition). This requires quite some time and puts a heavy burden on the nursing staff. Although for the monitoring of some vital parameters, there exist systems for more automated monitoring, the latter is not generally widespread, since it requires additional components and measuring/monitoring systems. Also during home therapy, patients may be asked to monitor vital parameters by performing different measurements. Patients then typically note these down on a list, which is checked by a medical doctor when he visits the patient or in a digital interview. Alternatively, the parameters are checked by nursing staff.

In case of oxygen therapy delivery it is for instance unknown if the patient holds his nasal cannula. Does the patient sometimes breathe through the mouth? Has the patient been breathing harder, weaker, faster or slower? Have the breathing parameters remained stable? And so on. There is no certainty for the doctor that the prescribed dose has actually been administered. In addition, it is unknown whether the patient does take in oxygen when the caregiver leaves the room. Even at night and when the patient is asleep, it is not known what happens and whether the correct dose was taken. These are all examples considering oxygen therapy, similar examples can be given considering other forms of therapy where it is of interest to follow up the same vital parameters. Because there is no continuous follow-up possible for less critical patients, it can be difficult to make good decisions for adapting therapy to the patient's needs. This has consequences for the well-being of the patient, the effectiveness of the treatment and ultimately the general length of stay in the hospital.

The use of gases in medical applications is widespread. One of the gases often administered to patients for medical reasons is oxygen. In view of the fact that administering oxygen is often essential for preventing damage to tissue, avoiding life-threatening situations or saving a patient from a life-threatening situation, hospitals distribute oxygen using a pipeline network up to the bed of nearly each patient. Furthermore, oxygen therapy is also widespread for homecare patients, making use of pressurised oxygen bottles and or oxygen concentrators. The conventional form of administering oxygen through a pipeline network is in a continuous manner and lacks any means for monitoring the therapy delivered. A flow meter can be set to the flow rate that the patient needs (1 I/min up to 15 I/min continuously or higher). This way the oxygen flows continuously from the source to the patient via a nasal cannula during inhaling and exhaling. In hospitals, the flow meter is plugged into the low-pressure oxygen socket (in Europe usually between 3.6 and 5.5 bars) on the wall behind the patient's bed. A moisturizer can be attached to the bottom side to prevent drying up of the nasal mucous membrane. Similarly at home, a flow meter can be connected to the pressurised oxygen bottle, whereby typically a pressure reducer is introduced in between the flow meter and the pressurised oxygen bottle or is integrated in the bottle or the oxygen concentrator.

The lungs can only utilise the first phase of inhalation to exchange oxygen with the blood circulation. It is clear that oxygen can no longer be 'consumed' during the expiration phase (exhalation). However, during the last phase of inhalation too, only the large bronchial tube that does not participate in the diffusion process of oxygen is filled. Gas valves for on-demand supply of medical gases are based on this principle: oxygen is purposefully administered during the first phase of inhalation by the patient. This way a maximum oxygen intake is achieved and the oxygen that is not used is minimised. This results in oxygen conserving without impacting the oxygen therapy. Various gas valves have been developed during the past 20 years which are based on the principle of discontinuous oxygen administration in order to enable the use of light weight and smaller, sometimes portable, concentrators or smaller more lightweight cylinders for increasing the mobility of patients and/or to increase the autonomy of recipients. After all, the use of gas valves for mobile patients conserves oxygen, which results in a lower consumption and consequently creating longer autonomy or being able to give higher therapy levels while the oxygen concentrating capacity of a concentrator is restricted by its physical limits, i.e.3 to 5 times longer than the autonomy achieved with continuous administration or being able to make less energy consuming, more compact, lightweight and more efficient devices for concentrating oxygen out of ambient air.

Most gas valves are based on a regulating system in which nasal inhalation activates the gas valve. The underpressure activates and opens an oxygen valve through which an oxygen pulse (aka bolus) is generated. Detecting the inhalation and supplying the oxygen occurs by means of a nasal cannula. A nasal cannula can be a one-channel system (detection and supply occur through the same channel) or a two-channel system in which one channel is used for detecting the inhalation and the other channel is used for supplying the oxygen.

There is still room for improvement.

### Summary of the invention

It is an object of embodiments of the present invention to provide good methods and systems for identifying a condition, e.g. a medical condition or another breathing cycle influencing condition, of a user. In some embodiments, such methods and systems advantageously may make use of a gas valve system for delivering a gas to a user. It is an advantage of such embodiments of the present invention that gas valve system for delivering a gas to a user are widely spread and therefore readily available. It is an advantage of embodiments of the present invention that in this way identification of a condition of a user can easily be done for a large group of users. Alternatively or in addition thereto, the method or system is adapted for obtaining breathing cycle information in any suitable way, such as for example as data input or using another system for obtaining breathing cycle information.

The present invention relates to a system being adapted for deriving information regarding a condition of the user, the system comprising a processor configured for obtaining information related to the breathing cycle, wherein the processor furthermore is configured for comparing the derived information related to the breathing cycle of the user with predetermined breathing cycle information representative of different conditions and for identifying, based on said comparison, a condition of the user. In some advantageous embodiments, the system may be a system for delivering a gas to a user or for assisting therein, such as for example a gas valve system, although embodiments are not limited thereto. It is an advantage of such embodiments of the present invention that, aside from providing medical gas therapy, the system also allows for determining a medical condition of a user.

The methods and systems can be based on information related to the breathing cycle captured at the gas valve system, information related to the breathing cycle captured from another external or internal device such as for example a wearable or another system, or can be based on information related to the breathing cycle that is received as data input directly in the system. It is an advantage of embodiments of the present invention that the identification and/or the evolution of the condition of a user, such as for example a medical or other breathing cycle influencing condition, can be followed up and/or evaluated using the system. It is an advantage of embodiments of the present invention that the evolution of a certain medical or other breathing cycle influencing condition, e.g. disease, can be followed up over time for a user. The latter may allow to generate advice such as for example a medical advice, therapeutic suggestions and/or suggestions for precautionary matters. It is an advantage of embodiments of the present invention that not only an evaluation of the current medical condition can be made, but also that a pre-symptomatic analysis of a condition can be obtained. The latter allows for pro-active care and treatment, where applicable.

Whereas it is an advantage that the system providing information regarding a condition of a user is based on a system for delivering a gas to a user or assisting therein, a medical device that is widely spread and therefore available for a large population of users, especially but not limited to patients, it is to be noted that delivery of gas is not strictly required during detection of the identification and/or evolution of the condition of the user.

The system may comprise a flow and/or pressure sensor configured to be in fluidic connection with a nasal cannula or breathing mask for a user, for sensing at least a negative pressure signal, and wherein the processor may be configured for deriving, directly based on said at least a negative pressure signal, information related to the breathing cycle.

It is an advantage of embodiments of the present invention that either information obtained from a wearable or another measurement system can be used that is communicated to the system and/or that information can be derived based on the system itself. In the latter case, advantageously the system itself provides the required information regarding the breathing cycle.

The condition may imply an effect on the breathing cycle. The condition may imply any of a medical condition or another breathing cycle related condition. The condition may imply a stage, phase, trend or evolution of the condition implies an effect on the breathing cycle. The medical condition can for example be - but is not limited to - a condition of pneumonia, a condition of COPD, a condition of Covid-19, a heart failing condition, .... Another breath cycle related condition can for example be - but is not limited to - a condition of sleeping apnea, snorring, .... Other conditions that can be determined can for example be whether a user is breathing through the mouth, through the nose, is talking, eating, walking, climbing stairs or has a deep or shallow breathing pattern.

Whereas in embodiments of the present invention reference is made to a condition, this may for example thus refer to a disease, a phase or stage or evolution of a disease, a breathing or sleeping event/trend or a phase or stage or evolution of such a breathing event/trend.

This can for example thus be related to conditions that are directly related to the respiratory system, but also to other conditions that indirectly have an effect on the respiratory system. For example, from studies it has been made clear that although a heart failure condition is more related to the vascular system than to the respiratory system, the breathing cycle of the patient experiencing a heart failure condition is influenced or affected in a particular manner. A further, non-limiting list of examples will be discussed further in the description.

The system furthermore may be adapted for combining the derived information related to the breathing cycle of the user with another parameter of the user. It is an advantage of embodiments of the present invention that multiple parameters can be combined for determining thereof a condition of the user.

It is an advantage of embodiments of the present invention that aside from merely identifying a certain disease or condition, the system also may provide information regarding the phase of the disease or condition the user is in or may provide information regarding the evolution of the condition.

The information related to the breathing cycle may be one or more of a breathing frequency or breathing intensity or a breathing profile during the breathing cycle or trends or events thereof or a delayed breathing.

The system furthermore may be adapted for obtaining one or more further vital parameters and wherein the processor is configured for also taking into account said one or more further vital parameters for identifying the condition of the user. The system may be adapted for obtaining said further vital parameters from other measurement systems, including but not limited to wearables.

The system furthermore may be adapted for obtaining further patient information and wherein the processor is configured for taking into account said further patient information for identifying the condition of the user.

The system may be adapted for communicating further patient information with an electronic patient file.

There may be communication by the system to the electronic health record, communication to the system from the electronic health record or communication in both directions between the system and the electronic health record.

The further patient information may comprise one or more of age, gender, patient's physical parameters such as weight or length, medical information e.g. from the electronic patient file, a previously identified pathology or results from previous medical tests such as CT scan information, laboratory results of blood or other bodily fluid testing, ....

The processor may be configured for using any of artificial intelligence such as neural networks, machine learning, deep learning or pattern recognitions, predetermined algorithms or look up tables for identifying the condition of the user.

The predetermined breathing cycle information representative of different conditions may comprise breathing profiles representative for conditions or phases thereof associated with breathing problems or trends thereof.

The processor furthermore may be configured for providing a suggestion for change in therapy.

A change in therapy can for example be a change in medical gas therapy, although embodiments are not limited thereto. In one example, the change in therapy may also be a change to a department with more or less medical control (e.g. a switch between a high care, mid-care or low care department).

The suggestion for change in therapy may comprise one or more of a suggestion for the application of continuous or on-demand gas delivery, a suggestion for changing a frequency of monitoring and/or treating the patient, a suggestion for changing a flow rate of the gas delivery, a suggestion for switching between the medical department where the patient is treated, a suggestion for treatment by home care or in a hospital, a suggestion for changing a position of the user, a suggestion for changing in monitoring device, a suggestion for performing a medical intervention or a suggestion to a patient for performing a certain activity, a suggestion for treatment (such as for example a suggestion for medication or a suggestion of a dosis of medication) or a level of physical activity.

The system may be adapted for performing said suggestion for change in therapy in a closed loop system.

The system furthermore may be adapted for adding the obtained breathing cycle information and associated identified condition to a database storing the predetermined breathing cycle information representative of different conditions or for adding other information captured in or by the system.

In one aspect, the present invention also relates to a system for identifying a parameter of a user, the system comprising
a means for obtaining information related to a breathing cycle of the user,
processing means for determining at least one metric from the information related to the breathing cycle of the user.

The means for obtaining information may in some embodiments be a gas system collecting the information or any other type of sensor or a data input for receiving information collected through another, optionally external, means.

The system furthermore may comprise a processing means for evaluating the at least one metric and for deriving based on the evaluation a condition of the user.

The processing means for determining the at least one metric and the processing means for evaluating the at least one metric and for deriving the condition of the user may be implemented in a single processor or in a plurality of processors. The processing may be performed centrally at one place or in a distributed manner. It may e.g. be performed partly by cloud processing or on a central server, or it may be performed locally, e.g. in a system positioned close to the user.

The processing means for determining at least one metric may be configured or programmed for performing at least one or a combination of
identifying and ignoring, identifying and replacing, or identifying and removing bad quality data of the information related to the breathing cycle of the user,
normalizing the information related to the breathing cycle of the user, and removing noise from the information related to the breathing cycle of the user.
Identifying and ignoring or identifying and removing bad quality data may comprise one or more of ignoring or removing datapoints exceeding a hard threshold, ignoring or removing datapoints exceeding local variability thresholds, and ignoring or removing datapoints comprising missing values.

Normalizing the information related to the breathing cycle of the user may comprise normalizing the data based on user-specific historical data. The historical data may be data collected under predetermined conditions, e.g. under a predetermined protocol such as steady breathing, lying down, without speaking or breathing synchronous with an indicator on a user interface of the system.

Removing noise may comprise one or more of averaging or taking the median observed signal over a fixed historical time period, applying a low-pass filter reducing the presence of high-frequency components in the signal or locally decomposing the signal in its frequency and phase components, applying thresholding and recomposing the signal.

The processing means for determining at least one metric may be configured or programmed for classifying segments of the information related to the breathing cycle of the user. The processing means may be configured or programmed for applying either a segment-specific modeling technique depending on the classification of the segments or a general modeling technique. The general modeling technique may be implemented with a segment-specific parameterization.

The processing means for determining at least one metric may be configured or programmed for postprocessing the at least one metric by at least one of removing variability from the at least one metric or annotating the at least one metric with uncertainty estimates.

In still another aspect, the present invention also relates to a system for determining a metric related to a breathing cycle of the user, such as for example the vital parameter respiratory rate. The system comprises
- a first system for deriving, based on a negative pressure signal obtained from breathing of a user, a metric representative for information relating to the breathing cycle, such as for example the vital parameter respiratory rate,
- a further system configured for determining a metric representative for information related to the breathing cycle, such as the vital parameter respiratory rate, in an alternative way, and
- a processing means, e.g. processor, for presenting or combining the metric information from the first system and the further system.

The first system may be a gas delivery system comprising a flow and/or pressure sensor configured to be in fluidic connection with a guiding means for guiding a gas into the nose and/or mouth of a user, whereby the flow and/or pressure sensor is being configured for sensing at least a negative pressure signal.

Combining the metric information from the first system and the further system may for example be using information from the first system for calibrating the further system or vice versa. Which system is used for providing the calibration information may depend on the breathing conditions of the user. Combining the metric information may also include validation of data of the first or the further system, using the other system.

Presenting the metric information from the first system and the further system may depend on for example the breathing conditions of the user. The system that is most sensitive under certain breathing conditions may be used and the corresponding measurement data may be represented.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### Brief description of the drawings

FIG 1 illustrates an exemplary system for determining a metric based on breathing cycle information of a user, according to an embodiment of the present invention.
FIG. 2 illustrates a flow diagram of an exemplary method for determining a condition of a user based on breathing cycle information of a user, according to an embodiment of the present invention.
FIG. 3 illustrates a flow diagram of an exemplary method for processing raw data for the deriving of a metric from breathing cycle information of a user, according to an embodiment of the present invention.
FIG. 4 illustrates a flow diagram of an exemplary method for pre-processing raw data, according to an embodiment of the present invention.
FIG. 5 illustrates a flow diagram of an exemplary method of modelling data for deriving a metric from breathing cycle information of a user, according to an embodiment of the present invention.
FIG. 6 illustrates a flow diagram of an exemplary method of post-processing modelled data for deriving a metric from breathing cycle information of a user, according to an embodiment of the present invention.
FIG. 7 and FIG. 10 illustrates different data collection conditions and identification thereof, as applied in embodiments according to the present invention.
FIG. 8 and FIG. 9 illustrates different steps of an exemplary method for converting raw data related to breathing cycle information into a metric, according to an embodiment of the present invention.
FIG. 11 illustrates a system combining two metric determining devices, according to an embodiment of the present invention.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### Detailed description of illustrative embodiments

The present invention will be described with respect to particular embodiments and with reference to certain drawings but the invention is not limited thereto but only by the claims. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.
In the below description, as indicated above in some embodiments - although embodiments are not limited thereto - the system may be a gas valve system adapted for controlling the flow of a gas for a user, such as for example oxygen gas for oxygen gas therapy. Alternatively, the gas involved may for example be pressurized air, such as for example in sleep apnea applications. The gas valve system may be for use in hospital, for use in care institutions, for use in home care applications or may be for mixed use. The gas valve system may be merely for sensing and measuring, without delivery purposes as in a "measuring" or "no flow" mode, for continuous flow and/or may be for on demand use. Where reference is made to on demand gas valve systems or gas valve systems operated in on demand mode, reference is made to gas valve systems wherein no continuous gas delivery is provided but wherein gas delivery is based on a trigger, e.g. breathing inhalation of the user or based on other measured breathing cycle information. In some embodiments according to the present invention, the gas valve system may be as described for example in international patent application WO2012/153293, although embodiments are not limited thereto. The gas valve system may according to some embodiments be based on only mechanical components or may be based on a combination of mechanical and electronical components. Particular features such as for example the amount of gas that can be delivered (e.g. at least up to 10 liter/min., or at least up to 15 liter/min. or at least up to 25 liter/min. or at least up to 40 liter/min.) may be as described in WO2012/153293, although embodiments are not limited thereto. In a first aspect, the present invention relates to a system adapted for deriving information regarding a condition of the user. The system may in some embodiments be a gas valve system for delivering a gas to a user or for assisting therein, although systems are not limited thereto. As indicated above, the conditions of the user that can be identified typically are conditions having an effect on the breathing cycle, since the system uses breathing cycle information for identifying the condition of the user. Such a condition may express a stage, phase, trend or evolution of a medical or other condition of a user.

The system according to the present invention comprises a processor configured for obtaining information related to the breathing cycle. Obtaining information related to the breathing cycle can in some embodiments be obtaining information related to the breathing cycle from an external device, such as for example a standalone measurement device or a wearable. Alternatively, the system may be adapted for itself deriving breathing cycle information. The system then is adapted for performing measurements and deriving therefrom breathing cycle information.

The processor furthermore is configured for comparing the derived information related to the breathing cycle of the user with predetermined breathing cycle information representative of different conditions and for identifying, based on said comparison, a condition of the user.

By way of illustration, standard and optional components of the system will now further be discussed by way of an exemplary system.

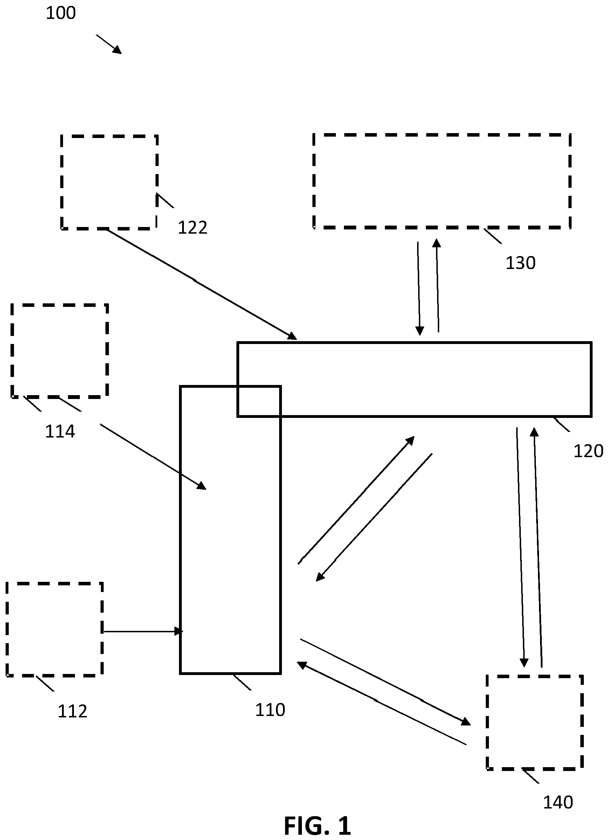

FIG. 1 illustrates an exemplary system according to embodiments of the present invention. The system 100 shown in FIG. 1 comprises a processor for performing processing in determining a metric from the breathing cycle information and in determining a condition of the user from the metric. In the present example, the processing is performed partly in a local processor 110 and in a cloud processor 120. The processing thus may be performed in a distributed manner at different locations, e.g. in a local processor and a cloud processor, but alternatively, may be performed at a single location, e.g. either in a local device or in cloud computing, in a single or in a plurality of processors. The system 100 is configured for obtaining breathing cycle information. The latter may be through an input port, or the system may comprise a breathing cycle data generation device 112. The breathing cycle information may be enriched with other information. Such information may be obtained via a device configured for obtaining first additional information 114, such as for example from external sensors, measurement systems, wearables, from user input, etc. Such information may for example be information regarding the capturing of the breathing cycle information such as for example a position the user is in, whether the user is performing a particular action at that moment in time, another parameter, such as for example a vital parameter of the user, etc. According to some embodiments, additional information also may be delivered to a cloud component. The latter may for example be performed by a device 122 obtaining second additional information. Such device 122 may be an external sensor, a measurement system, a wearable, or may be configured to provide user input, etc. In some embodiments, information also may be provided from an electronic patient file, i.e. through communication between the current system 100 and the electronic patient file system 130. In some embodiments, the system furthermore may be equipped with a module 140 that is configured for providing information, e.g. regarding the derived metric and/or regarding the derived condition of the user, to the medical staff and/or is configured for receiving information or control commands for the system from the medical staff.

In one embodiment, the exemplary system 100 or more particularly the breathing cycle information generation device 112 comprises a connecting element for connecting a nasal cannula or breathing mask or any other gas guiding means for guiding gas to a nose or mouth of a user to the system, for providing a fluidic connection to the patient. The connecting element may be referred to as a lead-through (also referred to as an outlet) for connecting the system to lead the generated gas flow to the patient. A classic example of such a system is a nasal cannula. A lead-through can be a one-channel or a two-channel lead-through (e.g., for connecting a one-channel or two-channel nasal cannula). A two-channel lead-through has two channels in which one channel is used for supplying the medical gas and the other channel is used for detecting the nasal inhalation at a specific moment. The lead-through can have a fitting connection for nasal cannulas available on the market for supplying medical gases to patients. Lead-through can have a specific geometry in a specific embodiment in order to correspond to a specific geometry of the system's connection that supplies the generated gas flow to the patient (e.g., a nasal cannula with a connection of a specific geometry). This will prevent a situation in which the system supplying the generated gas flow to the patient is connected by mistake to the wrong pipeline (e.g., a compressed air pipeline) or mistakes between in and outlet of homecare devices that have to be coupled to a distance source of gas at one side and to the patient at the other side.. In some embodiments, the system can be adjusted to operate with a two-channel nasal cannula and the system used for detecting the nasal inhalation at a specific time can be used to initiate the gas pulse. In other words, the pulse operation can be regulated on the basis of the detected signal. In other embodiments, the valve is adjusted to operate with a one-channel nasal cannula and the pulse operation is initiated by creating underpressure in the one channel. Similarly, the system may be adapted for operation with another guiding means for guiding gas into the nose or mouth of a user, such as for example a breathing mask like for example a nose mask, a mouth mask, a combined nose and mouth mask, a nose-pillow mask, a hybrid mask a face mask, a nasofaryngaele catheter, a transtracheal catheter, a partially rebreathing mask, a non-rebreathing mask, a high flow nasal cannula, a venturi mask, etc. The present invention is not limited to a particular system for providing a gas, e.g. medical gas, to a user.
In the exemplary system, the system furthermore may comprise a flow and/or pressure sensor in fluidic connection with the nasal cannula or breathing mask, for sensing the nasal or mouth inhalation as described above. Typically, this inhalation is sensed, when the nasal cannula or breathing mask is connected to the system, by at least a negative pressure signal. It is to be noted that aside the negative pressure signal, also positive pressure signals may be detected. In at least some embodiments, a full recording to the pressure during the breathing cycle is performed. Whereas reference is made to the pressure, alternatively or in addition thereto also the flow of the gas may be recorded. Embodiments of the current invention are suitable preferably for patients, e.g. persons or animals, with spontaneous breathing.
The flow and/or pressure sensor typically may correspond with features of electronic systems for delivery of medical gasses allowing on demand delivery of medical gas. Such systems do not continuously provide medical gas, but provide medical gas during the proper moment in time of the breathing cycle when the patient optimally benefits from the medical gas delivery. Such systems typically are developed to conserve medical gasses. The type of flow and/or pressure sensors that are used may be any type of flow and/or pressure sensor having sufficient sensitivity to accurately measure the breathing cycle of the patient. In some embodiments, the sensitivity should be sufficiently good to detect a positive and/or negative pressure signal in the range between 0.005mm H2O and 50mm H2O, e.g. between 0.1mm H2O and 10mm H2O, for triggering the delivery of medical gas when such a negative pressure is triggered. In some embodiments, the sensitivity should be sufficiently good to detect a positive and/or negative pressure signal for sensing delivery between 0.005 and 1.5 bar, e.g. between 0.01 and 1.0 bar. The pressure sensor or flow sensor may be adapted for deriving a pressure signal at predetermined moments in time, during at least one full breathing cycle, during a plurality of breathing cycles, etc. It may be adapted for deriving a pressure signal as function of time during at least one full breathing cycle or during a plurality of breathing cycles. The components of the system adapted for delivery of gas, on demand or continuous, may in some embodiments be equal to or similar to the system as described in international patent application PCT/IB2012/05329.
The system in some embodiments thus is adapted to derive information related to the breathing cycle, directly based on at least a negative pressure signal. Where in embodiments of the present invention reference is made to directly based on at least a negative pressure signal, reference is made to the fact that there is a direct link between the pressure signal sensed and the information regarding the breathing cycle. The latter is advantageous since e.g. whether or not a pulse of medical gas is created is irrelevant for determining the information regarding the breathing cycle. In some embodiments, the information of the breathing cycle may be determined based on a predetermined value of negative pressure sensed, based on a full pressure signal sensed over the full breathing cycle, .... The information regarding the breathing cycle comprises one or more of a breathing rate, a regularity or irregularity in the breathing cycle or in the breathing rate or a number of breaths per minute. It furthermore may comprise e.g. an intensity of breathing. The system may be adapted for continuously deriving the information regarding the breathing cycle or for deriving such information at predetermined moments in time, at a fixed frequency, when triggered by medical or nursing staff, etc.

In an alternative embodiment, the system does not derive the breathing cycle information by measurements performed in a gas valve system itself, but the breathing cycle information is obtained as data from an external device, such as for example a measurement system, optionally a wearable. A number of measurement techniques and corresponding devices are known for deriving breathing cycle information. A first exemplary measurement technique is an indirect measurement technique referred to as electrode-based impedance pneumography. The technique can make use of similar or the same electrodes as used for measuring an electrocardiogram and the corresponding heart rate. A second example is an optical technique referred to as "end-tidal CO2". End tidal CO2 can e.g. be measured using a capnometer which features a small plastic tube that is inserted in the patient's mouth. With each breath, the tube collects expelled CO2. The CO2 is then optically measured. A third exemplary measurement technique that can be used is based on photoplethysmogram detection measured from the patient's index finger during a conventional measurement of the patient's oxygen saturation. A fourth technique may be the manual detection of breathing information by medical personnel. A body-worn monitor for measuring the respiratory rate based on a fifth technique is described in European patent EP2560550. The obtained data is in this alternative embodiment typically obtained as data via a data input port, in communication with the external device. Overall, breathing cycle information may be derived in any suitable manner. For example, respiratory rate as well as other breathing cycle information may be derived for example based on manual or assisted counting, determination based on exhaled breathing, determination based on thoracic efforts, determination based on respiratory sounds, determination based on a light signal and determination based on indirect effects on the cardiovascular physiology or blood flow.

According to embodiments of the present invention, both for systems deriving the breathing cycle information based on measurements performed by the system or received as data from an external device, the processor furthermore is configured for comparing the derived information related to the breathing cycle of the user with predetermined breathing cycle information representative of different conditions and for identifying, based on said comparison, a condition of the user. The processor may for example make use of predetermined algorithms or look up tables, but alternatively also may make use of artificial intelligence such as neural networks, machine learning, deep learning or pattern recognitions. The breathing cycle information may be a respiratory rate, full cycle information regarding the breathing cycle or other information. The respiratory rate has been identified in literature as being representative for certain conditions of the patient. A non-limiting list of examples is given below. For example, an increased respiratory rate may be an indication of acute myocardial infarction, of pneumonia, of a pulmonary embolism, of aspiration, of haemorrhage or of metabolic acidosis. A decreased respiratory rate may be an indication of a drug intoxication, of hypercapnia, of an intoxication or of a head injury. Some more explicit examples are described below. In one example, as described by a study in 1993 by Fieselmann et al. in J. Gen. Intern. Med. 1993 vol 8 pp354-360, the respiratory rate can be used for predicting cardiopulmonary arrest for internal medicine patients. In another example, as described by Subbe et al. in Anaesthesia 2003 vol 58 pp797-802, relative changes in respiratory rate can be used for discriminating between stable patients and patients at risk. In yet another example, Goldhill et al. described in Anaesthesia 2005 vol 60 pp547-553 how the respiratory rate can be used as an early warning score (EWS) for ward patients. In still another example, Cretikos et al. described in Resuscitation 2007 vol 73 pp62-72 how the respiratory rate could be used to predict an upcoming serious adverse event on the general wards of a patient before this event occurred. Other examples of conditions that can be determined based on the breathing pattern may include hyperventilation syndromes, periodic deep sighing, forced abdominal expiration, thoracoabdominal asynchrony, thoracic dominant breathing, etc. Also during a COVID-19 infection, the breathing pattern of a patient shows particular features that allow to identify a state of the patient. According to some embodiments, the processor may be adapted for performing an identification of the condition according to embodiments as described by the second aspect.

The processing may be performed by a single processor or by a group of processors. The processor may be physically located at the same position of the actual valve controlling the gas delivery, but alternatively also may be positioned at a remote location and connected with the other parts of the system via a data transmission means.

Transmitting means for transmitting data between the different components are known to the person skilled in the art. They may rely on wired or wireless transmission. They may make use of conventional protocols or specific designed protocols. Typical protocols that may be applied are internet protocols, Bluetooth protocol, file transfer protocols, TCP and UDP-based protocols, through web services, etc. One particular example, embodiments of the present invention not being limited thereto, is communication according to a Bluetooth 4 protocol.

The processor may have an output means providing information regarding the identified condition of a user of the gas valve, for example, to the patient and/or the caregiver. The communication with the patient and/or the caregiver may use any suitable communication platform such as for example Bluetooth, internet, wifi, 4G or 5G, ...

According to some embodiments of the present invention, the system may be updated, either at predetermined moments in time, at regular times, or on demand. The system may be configured in modules, such that the possibility of identification of a particular condition, based on comparison with appropriate reference material, can be added during an update or upgrade of the system. Depending on where the processing of the system is performed, such an update or upgrade can be performed in a local device, in a cloud processor or at different positions, e.g. when distributed processing is performed.

It is to be noted that, aside from one metric based on breathing cycle information of the user, also further information may be used for deriving a condition. Such further information may be another metric based on breathing cycle information of the user, but also may be another metric regarding the user, such as for example another vital parameter or another parameter. Examples of other parameters that may be used can for example be oxygen saturation, heart rhythm, blood pressure, ... but e.g. also CT scan information, etc. It is an advantage of some embodiments of the present invention that by combining multiple parameters from a user, a more accurate determination or evaluation of a condition or the evolution thereof can be made. In some embodiments, such additional information also may be used for determining which conditions may be more relevant for the user under study.

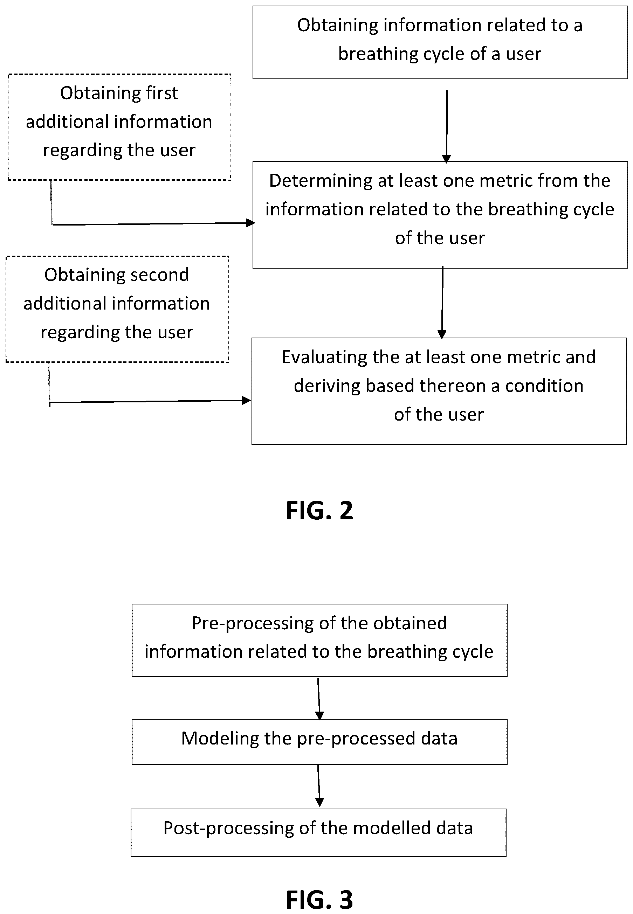

The system thus may in some embodiments be adapted for collecting, aside from vital parameters that can be derived based on measurements performed by the system itself or from a wearable or another external device, further data measured or collected by devices in the neighbourhood of the system and/or data from an electronic patient file. For example, the system may be equipped for collecting temperature data from a temperature monitoring device, heart rate from a heart rate monitor, oxygen level related data from an oxygen level monitoring device, blood pressure information from a blood pressure device, or combination of these data from plural vital parameter monitoring devices etc. Such data may be collected from wearables. Such data, once received in the system, may be stored separately or in combination with other information such that for example different parameters that measured at the same or approximately the same time are grouped, and may be either displayed in any suitable manner or transmitted to an external device. Aside from vital parameters, also information regarding the patient, information regarding the pathology as well as information regarding the medical gas therapy that is applied may be collected, processed, stored and/or transmitted by the system. Such information can, in addition to the breathing cycle information, be used for deriving the condition of the user. Such information may allow to further specify the condition, or to further distinguish between certain conditions. Further optional features may also be present, as illustrated further in the description, e.g. in the exemplary embodiments described.
In a second aspect, the present invention relates to a method for identifying a condition of a user, e.g. a medical condition of a patient, based on breathing cycle information.
According to embodiments of the present invention, the method comprises obtaining breathing cycle related information from the user. Such information may be obtained either as data input in the system or it may be directly derived by the system itself, e.g. when the system comprises or is part of a gas valve system. Thus, in some embodiments the step of obtaining breathing cycle information from the user may be a step of receiving breathing cycle related information from an external device, e.g. a wearable. In other embodiments, the step of obtaining breathing cycle information may be a step of deriving breathing cycle information by a gas valve itself. By way of example, such a step of deriving breathing cycle information may comprise a first sub-step, wherein pressure signal information is collected by the system, followed by a second sub-step wherein the pressure signal information is processed using signal processing. After the signal processing, in a further sub-step, evaluation of the processed signal is performed for determining corresponding breathing cycle information. This step may make use of pattern recognitions algorithms. In some exemplary embodiments , the processed signal may for example be compared with a scalable version of a breathing cycle curve. In this step, also signal disturbances such as snoring, coughing, talking and alike can be identified and excluded or identified as a sequence for which another algorithm is to be used to it for deriving the correct breathing pattern out of it.
In some embodiments, from the thus obtained breathing cycle information, a respiratory rate can be derived. The determined respiratory rate may be an actual respiratory rate, an average respiratory rate or even a trend of the respiratory rate. Alternatively or in addition thereto, also the strength of the breathing can be determined. The latter may for example be used for deriving whether the patient is breathing through his nose or through the mouth.
In a further step, the method comprises comparing the determined breathing cycle information, advantageously completed with other data such as other vital parameters, information from the electronic patient file such as pathology, age, gender, with reference breathing cycle information representative for certain conditions of a user. Based on this comparison, e.g. when certain parameters differ less than a predetermined threshold, a condition of the user can be identified or can be suggested to be taken in consideration.
In preferred embodiments, the obtained breathing cycle information in combination with the actual condition is added to the database with reference breathing cycle information representative for certain conditions of the user.
By way of illustration, embodiments of the present invention not being limited thereto, an exemplary method is described in FIG. 2. The method discloses a first step of obtaining information related to a breathing cycle of a user, followed by a step of determining at least one metric from the information related to the breathing cycle of the user. The method may also comprise obtaining first additional information regarding the user or regarding the information related to a breathing cycle of a user that is obtained. The exemplary method also illustrates the step of evaluating the at least one metric and deriving based thereon a condition of the user. It thereby may comprise an additional step of obtaining second additional information regarding the user, such as for example a further metric, medical data, information from the electronic patient file, etc. Further optional features may also be present, as illustrated further in the description, e.g. in the exemplary embodiments described, or expressing the functionality of the components of the systems as described in the other aspects.

In one aspect, the present invention also relates to a system and/or method for identifying a parameter of a user. According to embodiments of the present invention, the system comprises a means for obtaining information related to a breathing cycle of the user, which may be an input port for receiving data or which may be a system configured for collecting data from the patient. The system and method therefore does not need to include the actual measurement/collecting step and may only include obtaining the data related to a breathing cycle of the user, although in alternative embodiments, a measurement or collecting means or step may be included. The system furthermore comprises a processing means for determining at least one metric from the information related to the breathing cycle of the user. The system and/or method may, but does not need to, include a processing means for or step of evaluating the at least one metric and for deriving based on the evaluation a condition of the user. The processing may be based on predetermined algorithms or advantageously may make use of artificial intelligence. It is to be noted that processing may be performed in full on a local device, in full on a cloud processor or distributed, in a similar way as for processing steps described in the other different aspects. Optional features may - where applicable - be as described in the other aspects, and as described for examples shown.

In still another aspect, the present invention relates to a system and/or method for determining a metric related to a breathing cycle of the user, such as for example the vital parameter respiratory rate. The system comprises a first system for deriving, based on a negative pressure signal obtained from breathing of a user, a metric representative for information relating to the breathing cycle, such as for example the vital parameter respiratory rate. The system also comprises a further system configured for determining a metric representative for information related to the breathing cycle, such as the vital parameter respiratory rate, in an alternative way. It also comprises a processing means, e.g. processor, for presenting or combining the metric information from the first system and the further system. The system may be adapted for calibrating one system or data obtained therefrom with the other system or data therefrom, for presenting data of a selected system based on the conditions under which the breathing cycle information if obtained from the user, etc. The first system may be a gas delivery system comprising a flow and/or pressure sensor configured to be in fluidic connection with a guiding means for guiding a gas into the nose and/or mouth of a user, whereby the flow and/or pressure sensor is being configured for sensing at least a negative pressure signal, although the actual measurement and/or data collection means or step does not need to be part of the method or system.

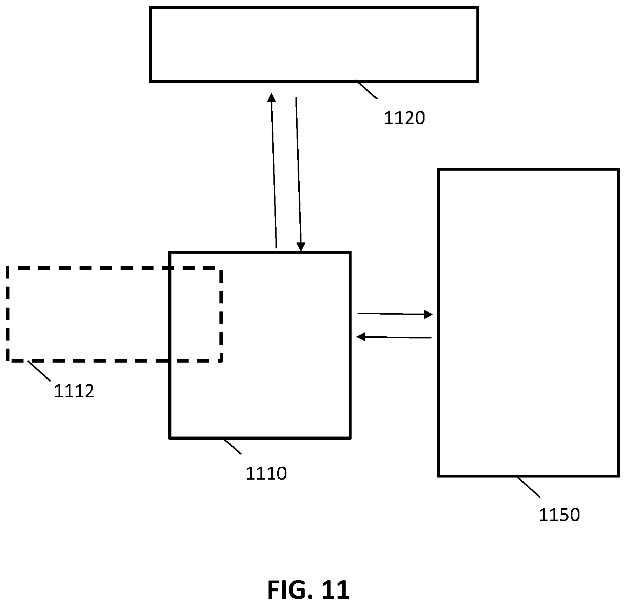

By way of illustration, embodiments of the present invention not being limited thereto, an example of a system for determining a metric is shown in FIG. 11. The system shows a local processor 1110 which e.g. can be used for part or all of the processing for determining a first metric from breathing cycle information, a breathing cycle data generation device 1112 and a cloud processor 1120 for performing part or all of the processing for determining the metric from the breathing cycle information, optionally doing pre-processing, modeling and post-processing, and/or for determining a condition based on at least the metric. The system also comprises a further system 1150 for determining the same metric. In one embodiment, the metric determined may be respiratory rate and the further system may be an optical system for determining respiratory rate from the movement of the chest of the user. Alternative systems, such as for example alternative systems for determining respiratory rate as described elsewhere in this description, also could be used. It is to be noted that processing may be performed in full on a local device, in full on a cloud processor or distributed, in a similar way as for processing steps described in the other different aspects. Optional features may - where applicable - be as described in the other aspects, and as described for examples shown.

By way of illustration, embodiments of the present invention not being limited thereto, an example of the processing performed on the raw data is shown below, illustrating how the raw data can be optimised for deriving breathing cycle information therefrom. These illustrate optional features of the system and/or method for determining a condition of a user or of the system and/or method for determining a parameter of a user.

In embodiments, processing of the raw data comprises at least one and preferably all of a preprocessing step, a modeling step and a postprocessing step. By way of illustration, embodiments not being limited thereto, an exemplary embodiment illustrating a system or method comprising a preprocessing step, a modeling step and a postprocessing step for converting raw data in data for deriving a metric is shown in FIG. 3.

Typically, the signal transformation steps may be operating on so-called windows of data. A data window contains a number of consecutive datapoints that are analyzed together. Any resulting breathing cycle information, such as for example a respiratory-rate (RR) related metric that is resulting from the processing of the raw data will therefor relate to all datapoints in the window, and consequently, to all timestamp associated to those datapoints. As such the length of the window (the window size) will control to what extent changes of the breathing cycle information, e.g. RR metric, can be localized in time. The larger the window size, in general the more accurate the average estimates of the RR metrics will be, but the harder it will be to pinpoint when exactly changes in RR metrics have occurred.

In some embodiments, the system is configured or programmed for performing pre-processing on the data, e.g. on the raw data. A number of preprocessing steps is provided as example herebelow. These steps not necessarily need to be applied all but may be applied all. Furthermore, these steps do not need necessarily to be performed in the order given by the different exemplary embodiments, but may. The pre-processing on the data may for example include one or more of (1) identify data of bad quality that should be discarded and/or removed (2) normalize data, i.e. remove subject-, device- and device operating regime-specific biases from the signal, e.g. making the resulting signal comparable (ceteris paribus) between subjects, devices or device operating regimes and (3) remove unwanted noise (variation in the pressure or oxygen release signals that is due to sensor measurement errors). For example, it can be advantageous to e.g. first remove noise from the incoming signal, before data are calibrated, although embodiments are not limited thereto.

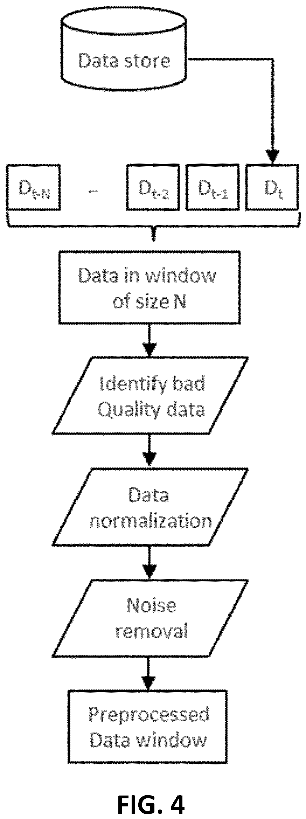

By way of illustration, embodiments not limited thereto, FIG. 4 illustrates a particular exemplary pre-processing system or method. In the exemplary embodiment, the pre-processing comprises obtaining the data, e.g. from a data store, and selecting therein data of a predetermined size, e.g. the data in a window of size N. The pre-processing in this example further comprises identifying bad quality data, a step of normalization of the data and a step of noise removal. After these steps are applied, this results in a preprocessed data window.

In some embodiments, an example of a preprocessing step that may be applied is the identification of bad quality data. Data labeled as bad-quality is not taking into account in any downstream processing. A number of methods may be used for assessing data quality and thus defining bad quality data. One method is using exceedance of hard thresholds. For example, all datapoint with a breathing pressure < 0 mbar and/or all datapoint with a breathing pressure higher than a predetermined pressure and/or all datapoint with oxygen release with a negative release rate and/or all datapoint with an oxygen release larger than a certain release rate may be considered bad data.

Identifying data of bad quality may in one embodiment include identifying data exceeding local variability thresholds. For example, data determined as bad data may be for example all datapoints present in part e.g. of a fixed-size set (e.g. data corresponding with a time period of e.g. 30s), of consecutive datapoints for which the standard deviation of the breathing pressure is smaller than a predetermined value or for example all datapoints in part e.g. of a fixed-size set (e.g. data corresponding with a time period of e.g. 30s), of consecutive datapoints for which the standard deviation of the breathing pressure is larger than a predetermined value. Data may also be determined as bad data when it relates to datapoints with missing values. Data may also be determined as bad data when it relates to datapoints for which the last good-quality datapoint lies more than a predetermined time period in the past.

In some embodiments, normalization and/or calibration can be performed, e.g. as follows. In some embodiments, subject-specific biases can be removed by applying one of the following strategies :
A first strategy that can be applied is to collect subject-specific data in a controlled context on first use of the breath information collecting device by a subject. A controlled context can for example consist of a predetermined sequence (a protocol) of actions either provided to the subject by a supervisor or by feedback provided by the breathing information collecting device user interface. Such a protocol should yield identical measurements for all variables transmitted, regardless of the subject, except for a subject-specific bias. The difference between the subject-specific measurements and a predetermined baseline (e.g. determined on a set of test subjects in an earlier phase) can be used to asses this subject-specific bias. Once this bias is known, it can be subtracted from any further measurements for the current subject in order to establish a subject-bias free signal. An exemplary protocol to assess subject bias can include:
- Steady breathing, lying down, without speaking for 1 minute
- Breathing synchronous with an indicator on the device user interface

Another strategy is to assume a systematic bias and use the mean and standard deviation calculated in the last period longer than a first predetermined amount of time, but less than a second predetermined amount of time in the past to normalize new datapoints by for example first subtracting this calculated mean followed by dividing by the calculating standard deviation.

Another possible strategy is applying the previous strategy, but use datapoints from the last recorded period where the subject was breathing steadily, without taking any other actions. This requires the classification of each datapoint in either steady breathing or not steady breathing. Below we provide a way to perform this segmentation.

Another way of normalization and/or calibration may be to characterize and remove device-specific and operation-specific biases in a way similar to subject-specific bias removal recording breathing pressure when
- the subject is not using a gas delivery system and thus no oxygen is supplied
- the subject is not using a gas delivery system and the gas delivery system cycles through a predefined sequence of operations for example switching between operation modes or releasing oxygen pulses with increasing length and flow rate.

Breathing pressure measurements could be severely disturbed when oxygen is released. In some embodiments, this could be addressed by e.g. identifying when oxygen is released based on the oxygen flow measurements transmitted from the gas delivery device and discarding that part of the respiratory cycle that is impacted by oxygen release, or e.g. by reconstructing the original breathing pressure signal by using the oxygen flow measurements to estimate the bias in pressure measurements, followed by subtracting this bias from the measured pressure signal.

In some embodiments, another possible pre-processing step is applied according to some embodiments is noise removal. Noise, defined as measurement variability that cannot be attributed to the subject, subject actions, the breathing information collecting device or device operation, can be reduced or removed for example by one or more of the following : averaging or taking the median the observed signal over a fixed historical time period, or applying a low-pass filter that reduces the presence of high-frequency components in the signal (e.g. through the use of low-pass filters), or locally decomposing the signal in its frequency and phase components using for example windowed Fourier or online wavelet analysis, thresholding the high frequency components (e.g. all components with a frequency larger than a predetermined value (e.g. > 0.5 Hz) and recomposing the signal.

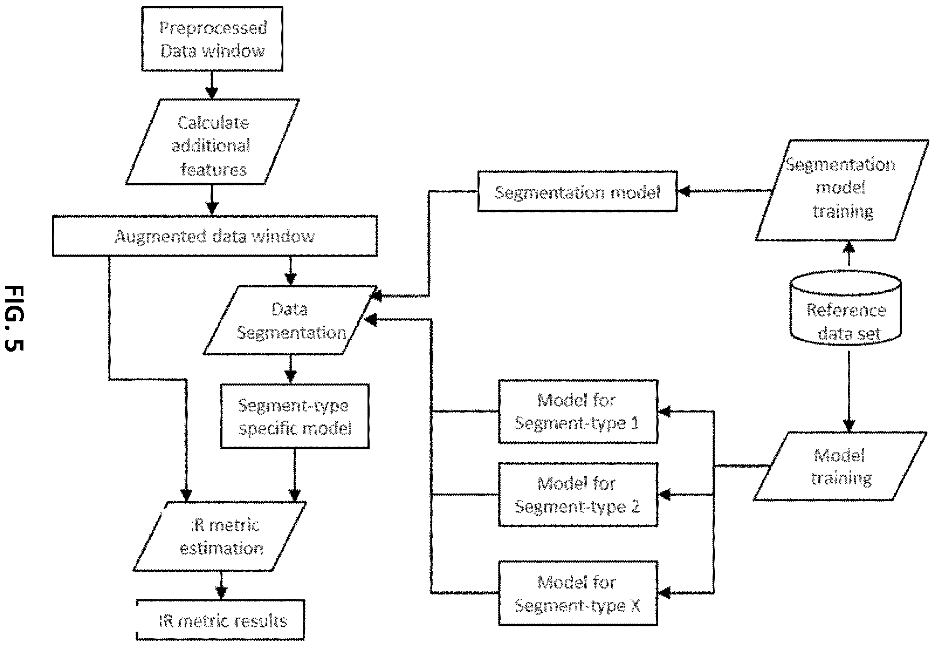

As indicated above, the processing of the data may comprise modeling. In some embodiments, modeling is performed and this may refer to one or more steps required to transform the preprocessed data signals into the desired target variable (e.g. respiratory rate). Such modeling may be either (1) uniform, indicating that an identical modeling strategy is applied to all parts of the signal, regardless of potential confounding factors, or (2) may be stratified (in a process called segmentation). The stratified approach implies that first an incoming datapoint is classified into categories (e.g., steady breathing, walking, talking, coughing, ...) after which a segment-specific modeling technique, or an identical modeling technique with a potentially segment-specific parameterization, is applied to the datapoint. Before any modeling takes places, we foresee to first supplement the preprocessed data with engineered features that are derived from the original data. By way of illustration, an example of modeling processing that can be performed in shown in FIG. 5.

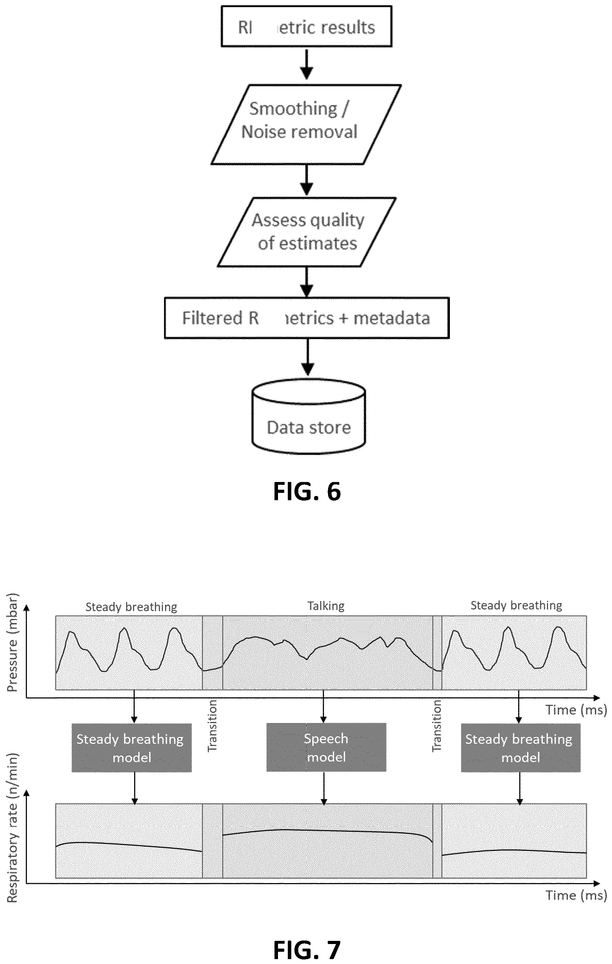

In some embodiments, optionally after modeling, the resulting quantitative or qualitative metrics are postprocessed in order (1) to further remove unwanted variability from the resulting signals, and/or (2) to annotate results with uncertainty estimates (the degree to which the models used are confident about their prediction). In this stage, data that could not be processed (for which no predictions could be made) because of data quality issues are labeled as such. By way of illustration, embodiments of the present invention not being limited thereto, an example of a postprocessing strategy is shown in FIG. 6.

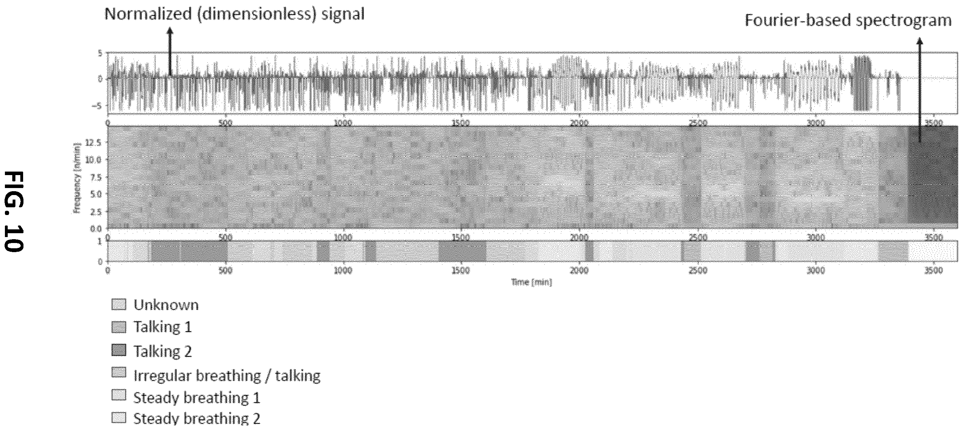

Further by way of illustration, embodiments not being limited thereto, the system may be configured to identify different collection conditions for collecting the data related to the breathing cycle. The system may for example identify that steady breathing was performed during the collection of the data related to the breathing cycle or for example identify that the user was speaking. The system may be equipped with an artificial intelligence module allowing to distinguish these - and optionally other - collection conditions. FIG. 7 illustrates by way of example, the determination of the respiratory rate from data recorded during steady breathing and data recorded during speaking. The artificial intelligence module may use a steady breathing model for data identified as collected during steady breathing and a speech model for data identified as collected during speaking. Implementing such analysis may result in a plurality of strategies : In some embodiments, metrics determined under different data collection conditions may be considered as different sources and therefore may help in data validation. In some embodiments, metrics determined under some particular data collection conditions may be considered of less or too low quality, and such data may be disregarded or removed. In some embodiments, metrics determined under some particular data collection conditions may be normalized differently compared to metrics determined under other particular data collection conditions, resulting in the fact that although they are collected under different data collection conditions, they can contribute to a same derived metric value. In FIG. 10, identification of a plurality of data collection conditions is illustrated. Whereas FIG. 7 illustrates the situation for steady breathing and for breathing during talking, similar situations are present under other conditions, such as for example snoring, doing exercises or other conditions.

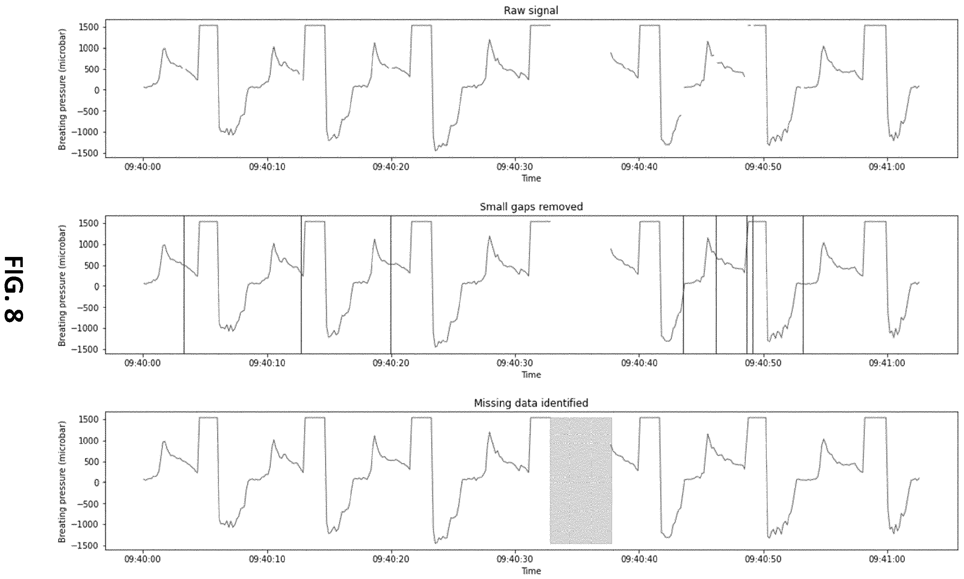

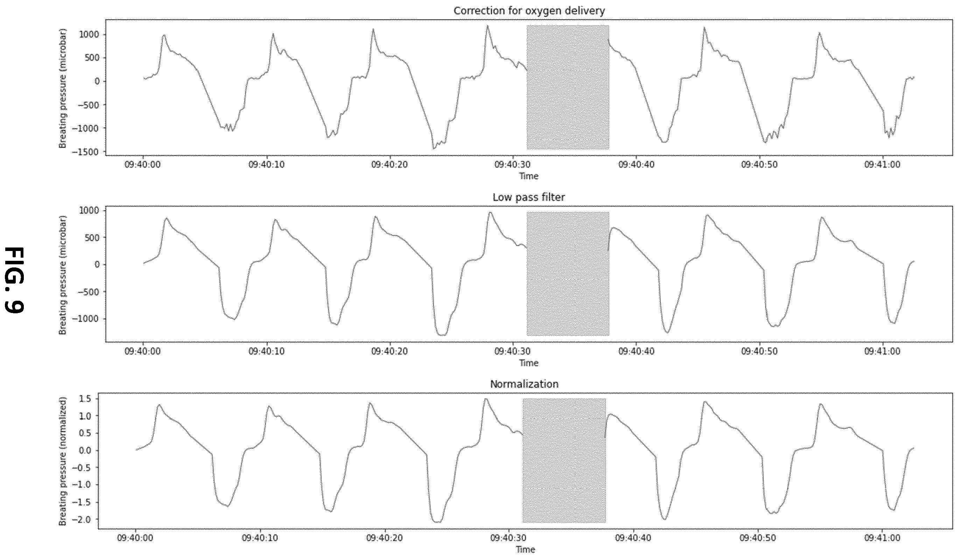

Also by way of illustration, a possible strategy for processing raw data related to breathing cycle is illustrated in FIG. 8 and FIG. 9. The upper portion of FIG. 8 illustrates the raw data as obtained from the user. The raw data in the present example corresponds with a pressure signal recorded using a gas delivery system, e.g. for providing oxygen therapy to a user. In a first step, small gaps occurring in the data are removed, as can be seen in the middle graph of FIG. 8. In a following step larger pieces of missing data are identified, as shown in the bottom graph of FIG. 8. The latter will be taken into account by determination of the metric. In a following step, a correction is performed for data collection conditions during which oxygen is delivered. This is illustrated in the top graph of FIG. 9. In a following step, a low pass filter is applied, as illustrated in the middle graph of FIG. 9. Finally also normalization is applied, as illustrated in the bottom graph of FIG. 9.

According to some embodiments, in methods and systems for identifying a condition of a user, the method is adapted or the system is configured for comparing the derived information related to the breathing cycle of the user - sometimes also referred to as a metric - with predetermined breathing cycle information representative of different conditions and for identifying, based on said comparison, a condition of the user. The predetermined breathing cycle information representative of different conditions may for example be breathing cycle information previously collected for users with known conditions using other methods and systems, statistical breathing cycle information obtained for a large population with particular conditions or breathing cycle information obtained during use of e.g. the artificial intelligence module .

As already indicated, the method or system may for example make use of predetermined algorithms or look up tables, but alternatively also may make use of artificial intelligence such as neural networks, machine learning, deep learning or pattern recognitions for comparing the derived information related to the breathing cycle of the user, e.g. the determined metric, with predetermined reference material.

One example of an algorithm that may be used is comparison of a value of the metric derived from the breathing cycle information with a value of the same metric for a population having a given condition. Particular rules may be applied, such as for example, a warning signal or a suggestion of the presence of a certain condition for a user may be given if the determined value of the metric is - depending on the metric and on the condition - e.g. higher, or lower or at least a predetermined amount higher, or a predetermined amount lower, or etc.

Another example is an automated evaluation of the deviation from certain reference information, e.g. based on an artificial intelligence module.

Whereas in the above often the example of respiratory rate is given, the information can also be another parameter derived from the breathing cycle information, such as for example breathing intensity, steepness of certain parts in the breathing cycle information, etc.

As also indicated earlier, for derivation of the condition, other parameters of the user also can be taken into account. For example, if oxygen saturation in the blood is taken into account, effects on the respiratory rate could for example be used more trustworthy to derive therefrom a condition related to the lungs, heart disease, sleep disease, infectious disease, clinical deterioration, pain, cognitive load, stress, postoperative adverse events or related problems and/or other conditions. Similarly, other parameters also may be indicative for certain other conditions.

## Claims

1. A system adapted for deriving information regarding a condition of the user, the system comprising
a processor configured for obtaining information related to the breathing cycle,
wherein the processor furthermore is configured for comparing the derived information related to the breathing cycle of the user with predetermined breathing cycle information representative of different conditions and for identifying, based on said comparison, a condition of the user.

2. A system according to claim 1, wherein the system comprises
a flow and/or pressure sensor configured to be in fluidic connection with a guiding means for guiding a gas into the nose and/or mouth of a user, the flow and/or pressure sensor being configured for sensing at least a negative pressure signal, and
wherein the processor is configured for deriving, directly based on said at least a negative pressure signal, information related to the breathing cycle.

3. A system according to any of the previous claims, wherein the condition implies an effect on the breathing cycle and/or wherein the condition is any of a medical condition or another breathing cycle related condition and/or wherein a stage, phase, trend or evolution of the condition implies an effect on the breathing cycle.

4. A system according to any of the previous claims, wherein said information related to the breathing cycle is one or more of a breathing frequency or breathing intensity or a breathing profile during the breathing cycle or trends or events thereof or a delayed breathing.

5. A system according to any of the previous claims, wherein the system furthermore is adapted for obtaining one or more further vital parameters and wherein the processor is configured for also taking into account said one or more further vital parameters for identifying the condition of the user.

6. A system according to any of the previous claims, wherein the system furthermore is adapted for obtaining further patient information and wherein the processor is configured for taking into account said further patient information for identifying the condition of the user.

7. A system according to the previous claim, wherein the system is adapted for communicating further patient information with an electronic patient file.

8. A system according to any of claims 6 or 7, wherein the further patient information comprises one or more of age, gender, patient's physical parameters such as weight or length, a previously identified pathology or results from previous medical tests such as CT scan information or laboratory test results from blood or other bodily fluids.

9. A system according to any of the previous claims, wherein the processor is configured for using any of artificial intelligence such as neural networks, machine learning, deep learning or pattern recognitions, predetermined algorithms or look up tables for identifying the condition of the user.

10. A system according to any of the previous claims, wherein the predetermined breathing cycle information representative of different conditions comprises breathing profiles representative for conditions or phases thereof associated with breathing problems or trends thereof.

11. A system according to any of the previous claims, wherein the system furthermore is adapted for combining the derived information related to the breathing cycle of the user with another parameter of the user.

12. A system according to any of the previous claims, wherein the processor furthermore is configured for providing a suggestion for change in therapy.

13. A system according to the previous claim, wherein the suggestion for change in therapy comprises one or more of a suggestion for the application of continuous or on-demand gas delivery, a suggestion for changing a frequency of monitoring and/or treating the patient, a suggestion for changing a flow rate of the gas delivery, a suggestion for switching between the medical department where the patient is treated, a suggestion for treatment by home care or in a hospital, a suggestion for changing a position of the user, a suggestion for changing in monitoring device, a suggestion for performing a medical intervention or a suggestion to a patient for performing a certain activity.

14. A system according to any of claims 12 or 13, wherein the system is adapted for performing said suggestion for change in therapy in a closed loop system.

15. A system according to any of the previous claims, wherein the system furthermore is adapted for adding the obtained breathing cycle information and associated identified condition to a database storing the predetermined breathing cycle information representative of different conditions or for adding other information captured in or by the system.
